(19) Europäisches Patentamt
European Patent Office
Office européen des brevets

(11) **EP 4 497 387 A1**

(12) **EUROPEAN PATENT APPLICATION**

(43) Date of publication:
**29.01.2025 Bulletin 2025/05**

(21) Application number: **24190504.1**

(22) Date of filing: **24.07.2024**

(51) International Patent Classification (IPC):
***A61B 5/346*** (2021.01)

(52) Cooperative Patent Classification (CPC):
**A61B 5/7217; A61B 5/346**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority:  **25.07.2023  US 202363528709 P
12.07.2024  US 202418771539**

(71) Applicant: **Biosense Webster (Israel) Ltd.
2066717 Yokneam (IL)**

(72) Inventors:
• **BOTZER, Lior
  2066717 Yokneam (IL)**
• **BAR-TAL, Meir
  2066717 Yokneam (IL)**
• **RAVUNA, Eliyahu
  2066717 Yokneam (IL)**
• **BIRNBOIM, Doron
  2066717 Yokneam (IL)**
• **EHRENFELD, Ilan Yaakov
  2066717 Yokneam (IL)**

(74) Representative: **Carpmaels & Ransford LLP
One Southampton Row
London WC1B 5HA (GB)**

(54) **FILTERING INTRACARDIAC ELECTROGRAMS IN THE PRESENCE OF PACING SPIKES**

(57)     A method for removal of a pacing artifact includes registering that a pacing signal has been applied to a pacing location in a heart of a patient. First and second electrodes are positioned respectively in first and second locations in proximity to the pacing location. First and second circuitries, having different, respective first and second transfer functions, are coupled to receive respective first and second ECG signals, generated in response to the pacing signal, from the first and second electrodes. First and second derived ECG signals are recorded from the first and second circuitries in response to the first ECG and second ECG signal. At least one of the first and the second derived ECG signals is analyzed in response to registering the pacing signal. In response to the analyzing, the pacing artifact is removed from the least one of the first and the second derived ECG signals.

EP 4 497 387 A1

Description

## CROSS-REFERENCE TO RELATED APPLICATION

This application claims the benefit of U.S. Provisional Patent Application 63/528,709, filed July 25, 2023, which is incorporated herein by reference.

## FIELD OF THE DISCLOSURE

This disclosure relates generally to electrophysiology, and specifically to signal acquisition during an electrophysiology procedure.

## BACKGROUND

During an electrophysiology procedure, for example during mapping of the electrical characteristics of one or more chambers of the heart, it may be advantageous to apply a pacing signal into the heart. Such a signal injects an electric current into the heart.

## BRIEF DESCRIPTION OF THE DRAWINGS

The present disclosure will be understood from the following detailed description, taken in conjunction with the drawings in which:

Fig. 1 which shows a catheter-based electrophysiology mapping and ablation system, according to an example of the present disclosure;

Figs. 2A and 2B are schematic block diagrams illustrating elements of the system used during a pacing procedure performed on a heart, according to an example of the present disclosure;

Fig. 3 shows schematic illustrations of a pacing artifact for balanced and unbalanced systems, according to an example of the present disclosure;

Fig. 4 is a block diagram illustrating the functioning of a signal analysis and pacing artifact removal block, according to an example of the present disclosure;

Fig. 5 is a flowchart showing steps performed by a processor to implement a DC correction in a DC correction block, according to an example of the present disclosure;

Fig. 6 shows graphs illustrating the operation of the DC correction block, according to an example of the present disclosure;

Fig. 7 shows a flowchart of steps performed by a processor in calculating the time point of an artifact reference time (ART), according to an example of the present disclosure; and

Fig. 8 is a flowchart showing steps to compute a fit function and to apply a decay function for baseline correction, according to an example of the present disclosure.

## DESCRIPTION OF EXAMPLES

OVERVIEW

During an electrophysiological procedure, pacing provides the professional performing the procedure with a means to characterize the myocardium. Pacing comprises injection of an electrical pulse, termed a pacing signal, into a location in the myocardium, and detecting intracardiac electrophysiological (EP) signals generated in regions of the myocardium other than the injection location. The intracardiac EP signals, herein, unless otherwise indicated assumed to comprise unipolar EP signals, are produced as a result of the action of the pacing signal on the myocardium. The pacing may be provided by an electrode of a catheter, and the unipolar EP signal detection may be performed by one or more other electrodes, which may be on the catheter or on other catheters, herein termed acquisition electrodes.

In addition to the detected EP signals, the pacing may produce electrical signals other than the EP signals on the acquisition electrodes. Such signals, herein termed pacing artifacts, are typically caused by changes in the potential field in the heart, due to the pacing signal, where the acquisition electrodes are placed. The EP signals, together with the artifacts are typically recorded on intracardiac electrocardiograms (ECGs) produced during the procedure, and the ECGs may be analyzed to achieve multiple clinical needs such as, for example, determination of local activation times (LATs) and wavefront mapping.

However, the presence of the pacing artifacts in the ECGs may lead to errors in the ECG analysis.

In examples of the present disclosure, the ECG analysis is assumed to comprise analysis of two unipolar EP signals acquired from two acquisition electrodes, together with analysis of the bipolar EP signal that is derived from the two unipolar signals. The two acquisition electrodes are respectively connected to an ECG recording system by respective transfer circuits. Each transfer circuit applies a respective transfer function to its received signal, so that two derived unipolar EP signals are provided to the ECG recording system. The derived bipolar EP signal that the recording system analyzes is formulated by calculating the difference between the two derived unipolar EP signals.

In some examples the two transfer circuits for the two electrodes are nominally the same, having components with the same nominal values arranged identically, so that both circuits have the same nominal transfer function. While the pacing artifact may be substantially reduced in the ECG analysis of the derived bipolar EP signal, deviations from the component nominal values, as well as the fact that the two acquisition electrodes do not acquire exactly the same pacing artifact because of their different spatial locations relative to the pacing source, and/or electrode differences themselves, typically leave a remaining pacing artifact in the derived bipolar signal. The remaining pacing artifact is herein termed a common transfer function pacing artifact.

In some examples the two transfer circuits for the two electrodes are different, so that each circuit applies a different transfer function to its received signal. In this case the pacing artifact, herein termed a different transfer function pacing artifact, is typically larger than the common transfer function pacing artifact.

Examples of the present disclosure provide an algorithm that analyzes derived EP signals to identify and compensate for the presence of both types of pacing artifact described above.

The artifact typically alters the signal acquired by the acquisition electrode in a number of ways, including altering a DC (direct current) voltage level of the signal and altering the slope of the baseline of the signal, i.e., imparting baseline wander to the signal. The algorithm corrects for any altered DC level and any baseline wander, and further corrects for any remaining alteration to the signal.

Applying the algorithm:

Minimizes the effect of the artifact on ECG presentations;
Eliminates incorrect bipolar voltage measurements that may be caused by the artifact; and
Eliminates false candidate annotations on an ECG.

SYSTEM DESCRIPTION

In the following description, like elements are identified by the same numeral, and are differentiated, where required, by having a letter attached as a suffix to the numeral.

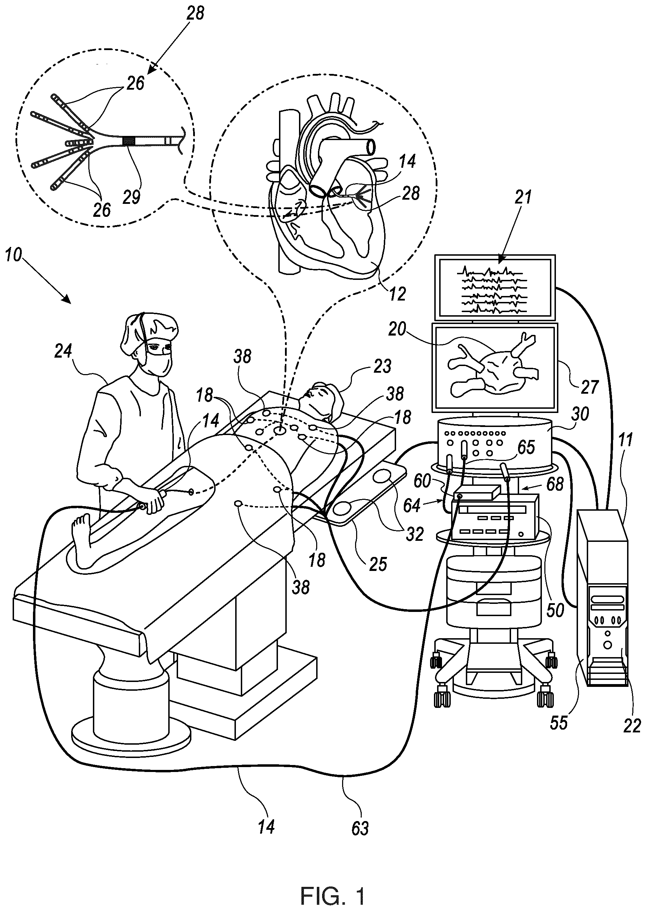

Reference is now made to Fig. 1 which shows a catheter-based electrophysiology mapping and ablation system 10, according to an example of the present disclosure. System 10 includes multiple catheters, which are percutaneously inserted by a physician 24 through the patient's vascular system into a chamber or vascular structure of a heart 12. Typically, a delivery sheath catheter is inserted into the left or right atrium near a desired location in heart 12. Thereafter, a plurality of catheters can be inserted into the delivery sheath catheter so as to arrive at the desired location. The plurality of catheters may include catheters dedicated for sensing Intracardiac Electrogram (IEGM) signals, catheters dedicated for ablating and/or catheters dedicated for both sensing and ablating. An example catheter 14 that is configured for sensing is illustrated herein. Physician 24 brings a distal tip 28 of catheter 14 into contact with the heart wall for sensing electropotentials (EPs) at a target site in heart 12.

Catheter 14 is an exemplary multi-spine catheter that includes multiple electrodes 26 distributed on the spines of the catheter. Catheter 14 may additionally include a position sensor 29 embedded in or near distal tip 28 for tracking the position and orientation of distal tip 28. Optionally and preferably, position sensor 29 is a magnetic based position sensor including three magnetic coils for sensing three-dimensional (3D) position and orientation.

Magnetic based position sensor 29 may be operated together with a location pad 25 including a plurality of magnetic coils 32 configured to generate magnetic fields in a predefined working volume. The real time position of distal tip 28 of catheter 14 may be tracked based on magnetic fields generated with location pad 25 and sensed by magnetic based position sensor 29. Details of the magnetic based position sensing technology are described in U.S. Patent Nos. 5,539,199; 5,443,489; 5,558,091; 6,172,499; 6,239,724; 6,332,089; 6,484,118; 6,618,612; 6,690,963; 6,788,967; 6,892,091.

System 10 includes one or more electrode patches 38 positioned for skin contact on a patient 23 to establish a location reference for location pad 25 as well as impedance-based tracking of at least some electrodes 26. For impedance-based tracking, electrical current is directed toward electrodes 26 and sensed at electrode skin patches 38 so that the location of each electrode can be triangulated via the electrode patches 38. Details of the impedance-based location tracking technology are described in U.S. Patent Nos. 7,536,218; 7,756,576; 7,848,787; 7,869,865; and 8,456,182.

A recorder 11 displays electrograms 21 captured with body surface ECG electrodes 18 and intracardiac electrograms (IEGM) that may be captured with electrodes 26 of catheter 14. Recorder 11 may include pacing capability for pacing the heart rhythm and/or may be electrically connected to a standalone pacer. As is described further below, in some examples pacing signals may be delivered through one of electrodes 26, or through another intracardiac electrode.

System 10 may include an ablation energy generator 50 that is adapted to conduct ablative energy to one or more of electrodes 26. Energy produced by ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) 30 is an interface configured to establish electrical communication between catheters, electrophysiological equipment, a power supply and a workstation 55 for controlling operation of system 10. Electrophysiological equipment of system 10 may include for example, multiple catheters, location pad 25, body surface ECG electrodes 18, electrode patches 38, ablation energy generator 50, and recorder 11. Optionally and preferably, PIU 30 additionally includes processing capability for implementing real-time computations of location of the catheters and for performing ECG calculations.

A system processing unit (SPU) 60, between PIU 30 and catheter 14, couples the PIU to electrodes 26 of the catheter, via cabling 65 between the PIU and the SPU and cabling 63 between the SPU and the electrodes. In some examples SPU 60 comprises respective front-end circuits 64, herein assumed to comprise a low-noise preamplifier, an analog-digital (A/D) converter, and filtration circuitry, that are applied to EP signals acquired by each electrode 26, prior to the signals being transferred to the PIU. In some examples, the SPU comprises respective modified front-end circuits 68, that, in addition to the circuitry of circuits 64, comprise further elements that enable electric current to be injected into a corresponding electrode 26, so that the electrode may be used for impedance-based tracking, as described above.

In examples of the present disclosure, the A/D converters referred to above are assumed to sample and digitize their analog signals at a rate of 1 ms.

Workstation 55 includes memory, a processor 22 with memory or storage with appropriate operating software loaded therein, and user interface capability. Workstation 55 may provide multiple functions, optionally including (1) modeling the endocardial anatomy in three-dimensions (3D) and rendering the model or anatomical map 20 for display on a display device 27, (2) displaying on display device 27 activation sequences (or other data) compiled from recorded electrograms 21 in representative visual indicia or imagery superimposed on the rendered anatomical map 20, (3) displaying real-time location and orientation of distal tip 28 within the heart chamber, and (4) displaying on display device 27 sites of interest such as places where ablation energy has been applied. One commercial product embodying elements of the system 10 is available as the CARTO™ 3 System, available from Biosense Webster, Inc., 31A Technology Drive, Irvine, CA 92618.

**Pacing by System 10**

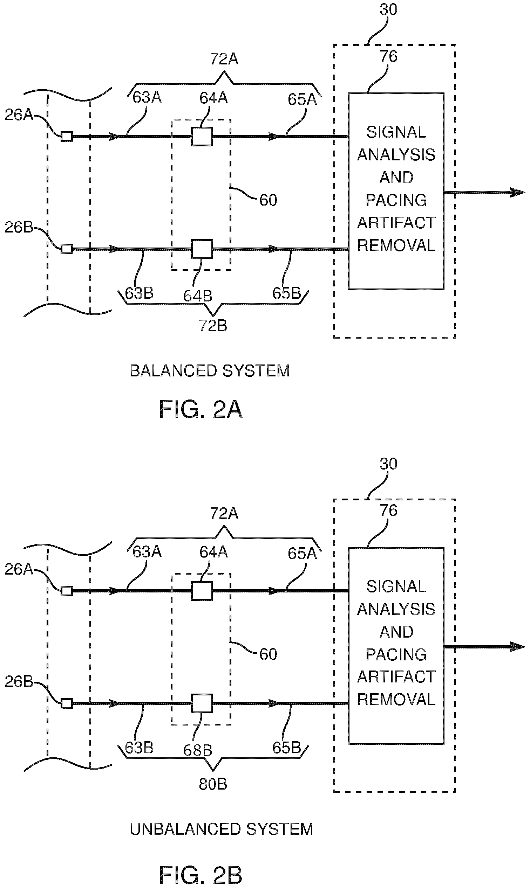

Figs. 2A and 2B are schematic block diagrams illustrating elements of system 10 used during a pacing procedure performed on heart 12 by the system, according to an example of the present disclosure. In the procedure, a pacing signal is injected into a region of the heart, and the resulting intracardiac EP signals, generated in response to the pacing signal, are acquired by electrodes on distal tip 28 of catheter 14 in contact with heart 12. The figures shows two such electrodes, electrodes 26A, 26B, herein by way of example assumed to be on a common spine of the distal tip. However, in other examples of the present disclosure, electrodes 26A, 26B are not on a common spine of distal tip 28. Electrodes 26A, 26B are herein also termed acquisition electrodes 26A, 26B. As is described below, the signals acquired by electrodes 26A, 26B are used to calculate a bipolar EP signal between the two electrodes.

In Fig. 2A each acquisition electrode 26A, 26B is connected, by respective transfer circuitry 72A, 72B, to a signal analysis and pacing artifact removal block 76. The structure and functionality of block 76 is described below, and by way of example elements of the block are assumed to be installed in PIU 30. The output of block 76 comprises resultant unipolar and bipolar signals, derived from the respective signals acquired by electrodes 26A, 26B, wherein the pacing artifact present on the acquired signals is removed. The resultant signals from block 76 may be provided to workstation 55, which may use the resultant signals, for example, for displaying and analyzing the corresponding IEGMs with the pacing artifact removed.

In Fig. 2A transfer circuitries 72A and 72B have the same physical components. Thus, circuitry 72A comprises cabling 63A coupling electrode 26A to front-end circuit 64A, front-end circuit 64A, and cabling 65A coupling front-end circuit 64A to block 76; and circuitry 72B comprises cabling 63B coupling electrode 26B to front-end circuit 64B, front-end circuit 64B, and cabling 65B coupling front-end circuit 64B to block 76.

The components of the two transfer circuitries illustrated in Fig. 2A are connected in the same configuration in the two circuitries, so that the figure illustrates a balanced system. However, while the physical components of the two transfer circuitries may have the same nominal values, it will be understood that since respective components of the circuits, such as their respective pre-amplifiers, are physically different, the actual electrical parameters associated with the components, such as their gains and/or impedances, are typically different.

Because of the differences in actual electrical parameters of the components of circuitries 72A and 72B, including differences of characteristics of individual electrode, a respective signal transfer function associated with each circuitry is different. The signal transfer function for a given transfer circuitry comprises a function defining the resultant signal generated by the transfer circuitry for a given input signal.

The input signal acquired by a given electrode 26 is composed of two sub-signals: a unipolar EP signal that is at least partly generated in response to the pacing signal injected into heart 12, and a pacing artifact also generated, by direct conduction, induction, and/or radiation, in response to the pacing signal. Because of the physical separation of electrodes 26A, 26B, the unipolar EP signal component acquired by electrode 26A is different from that of electrode 26B; similarly, the pacing artifact acquired by electrode 26A is different from the artifact acquired by electrode 26B.

In examples of the present disclosure, block 76 receives the resultant unipolar EP signals of circuitry 72A and of circuitry 72B, herein also termed the raw or derived unipolar EP signals of circuitry 72A and circuitry 72B, and initially calculates the difference in the derived signals, forming a raw bipolar EP signal. As is described below, block 76 is configured to remove the pacing artifact from the two raw unipolar EP signals, and from the raw bipolar EP signal, output by the block.

Reference is now made to Fig. 2B. Apart from the differences described below, the operation of the elements illustrated in Fig. 2B is generally similar to that of the elements in Fig. 2A, and elements indicated by the same reference numerals in both figures are generally similar in construction and in operation.

In contrast to the balanced system illustrated in Fig. 2A, the system illustrated in Fig. 2B is an unbalanced system. In Fig. 2B electrode 26A is connected to block 76 by transfer circuitry 72A, comprising, as is described above, cabling 63A, front-end circuit 64A, and cabling 65A. However, electrode 26B is connected to block 76 by transfer circuitry 80B. Transfer circuitry 80B comprises modified front-end circuit 68B, cabling 63B connecting circuit 68B to electrode 26B, and cabling 65B connecting circuit 68B to block 76.

As for the balanced system of Fig. 2A, in the unbalanced system of Fig. 2B block 76 receives the raw unipolar EP signal of circuitry 72A and the raw unipolar EP signal of circuitry 80B, and initially calculates the difference in the raw signals, forming a raw bipolar EP signal. As for the balanced system, block 76 is configured to remove the pacing artifact from the two raw unipolar EP signals, and from the raw bipolar EP signal, output by the block. Typically, the pacing artifact in the raw bipolar EP signal of the unbalanced system is larger than the pacing artifact in the raw bipolar EP signal of the balanced system.

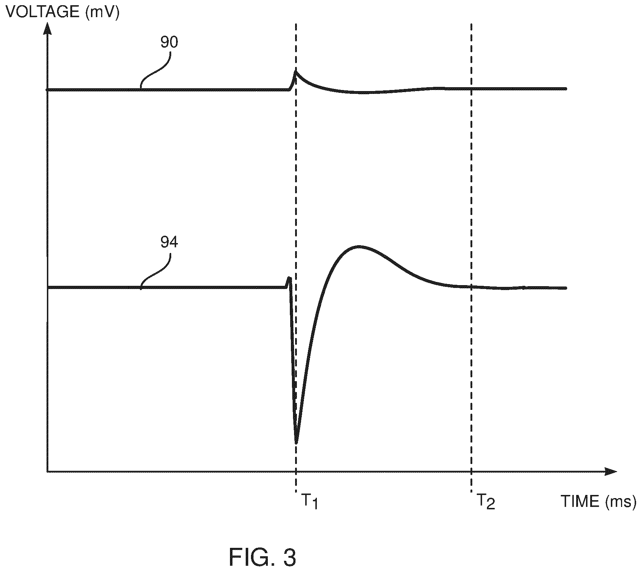

Fig. 3 shows schematic illustrations of the pacing artifact for balanced and unbalanced systems, according to an example of the present disclosure. A graph 90 is a voltage vs. time graph of the raw bipolar EP signal for a balanced system, as is initially calculated by block 76, and a graph 94 is a voltage vs. time graph of the raw bipolar EP signal for an unbalanced system. The pacing artifact occurs approximately between times $T_1$ and $T_2$, and as is apparent from the graphs, the pacing artifact for the unbalanced system, herein termed the different transfer function pacing artifact, is larger than that of the balanced system, herein termed the common transfer function pacing artifact.

In examples of the present disclosure, block 76 is configured to remove the pacing artifacts illustrated in Fig. 3, for balanced systems shown in Fig. 2A and for unbalanced systems shown in Fig. 2B, from the signal output by the block.

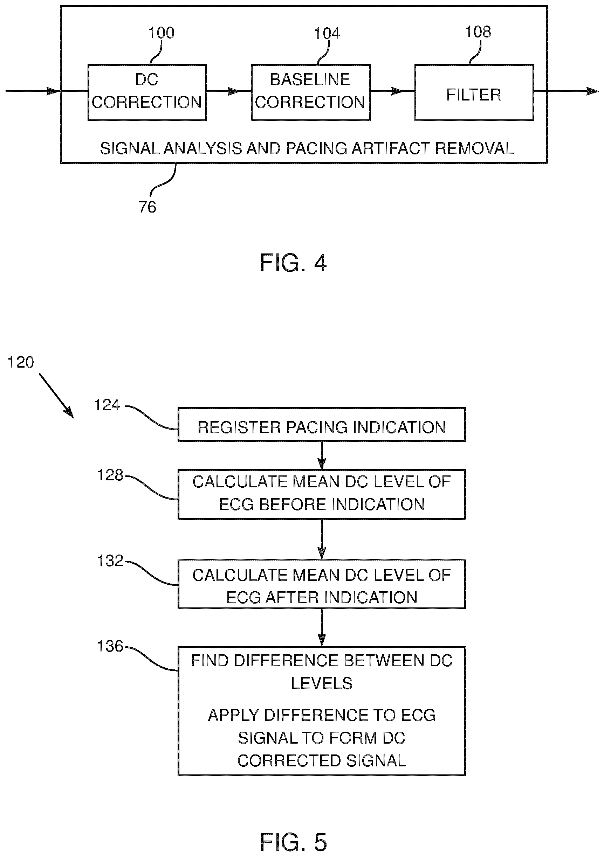

Fig. 4 is a block diagram illustrating the functioning of signal analysis and pacing artifact removal block 76, according to an example of the present disclosure. The pacing artifact that is impressed on each of the raw unipolar EP signals derived from electrodes 26, and on the raw bipolar EP signal formed from the unipolar signals, typically comprises the following elements:

A DC level change
A baseline slope change
A residual signal change.

For each of the three raw signals, i.e., two raw unipolar EP signals and a raw bipolar EP signal, examples of the present disclosure analyze the signals received from electrodes 26 to calculate the DC level change, and use the calculation to correct for the change, in a DC correction block 100. The DC corrected signal then transfers to a baseline correction block 104. In block 104 the baseline slopes are measured at two different times, and the measured slopes are used to generate a function that is applied to the baseline, to correct for the slope change. The baseline corrected signal transfers to a filtration block 108, wherein the residual signal change, while typically small, is removed by applying a filtering network to the baseline corrected signal.

The following sections describe the operation of each of the blocks in signal analysis and pacing artifact removal block 76. The operations are performed under overall control of processor 22.

DC Correction

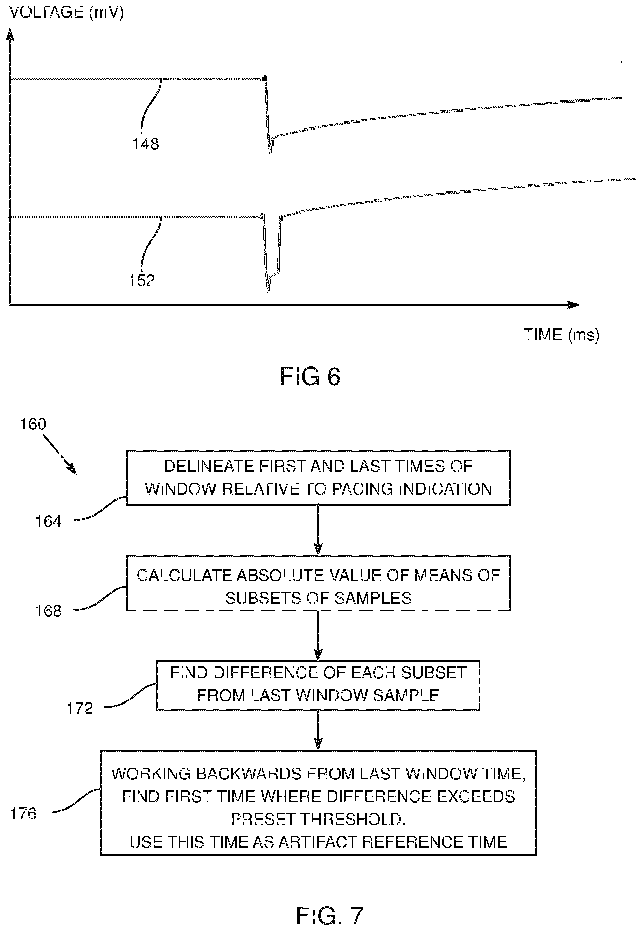

Fig. 5 is a flowchart 120 showing steps performed by processor 22 to implement the DC correction of block 100, and Fig. 6 shows graphs illustrating the operation of the block, according to an example of the present disclosure. The processor operates on sampled digitized signals to calculate the DC correction.

In an initial pacing registration step 124, processor 22 registers that a pacing signal has been injected into heart 12, and uses the time of registration as a pacing indication, also herein termed a fiducial time point. The processor also identifies the sample at the fiducial time point as a fiducial sample. The injection of the pacing signal typically generates a voltage on electrodes 26 of catheter 14 other than electrodes 26A, 26B, or on electrodes of another catheter that may be positioned in heart 12. The generated voltage is usually at least twice a typical maximum EP voltage generated by heart 12, so the processor is able to assume that this voltage is caused by the injection of a pacing signal.

The following description applies to the analysis performed on each of the three signals received by block 76, i.e., two raw unipolar EP signals and a raw bipolar EP signal.

In a prior DC level step 128, the processor measures a mean DC level of the raw EP signal over a preset prior time window before the fiducial time point. In one example the prior time window has a fixed width of 3 ms, between 5 ms and 2 ms before the fiducial time point. The time window bounds are typically selected to be as close as possible to the fiducial time point.

In a post DC level step 132, the processor measures the DC level at a dynamic time point within a post time window after the fiducial time point. In one example the post time window is between 10 ms and 20 ms after the fiducial time point, and the dynamic time point, herein also termed the artifact reference time (ART), within the window may be found, by an algorithm, described below with reference to a flowchart 160 in Fig. 7.

In a final step 136, the processor finds the difference between the DC levels of step 132 and step 128, and applies this difference to correct the raw EP signals, after the fiducial time point. The DC corrected signals formed in step 136 are transferred to baseline correction block 104.

Fig. 6 illustrates a DC correction applied to a raw bipolar EP signal, according to an example of the present disclosure. A graph 148 is a voltage vs. time graph of a raw EP bipolar signal prior to application of a DC correction, and a graph 152 is a voltage vs. time graph (translated parallel to the voltage axis for clarity) after application of the correction. As is illustrated by the graphs, the DC correction shifts the baseline, after an input signal comprising the pacing response and the artifact, so that the shifted baseline initiates at the same voltage level as the baseline before the input signal.

Reference is now made to Fig. 7, which shows a flowchart 160 of steps performed by processor 22 in calculating the time point of the artifact reference time (ART), according to an example of the present disclosure. In contrast to the preset time window described above, which uses preset time points, the ART is, as stated above, a dynamic time point which may change from signal to signal.

As is explained above, to correct for the presence of the pacing artifact, examples of the present disclosure apply a DC correction and a baseline correction. The ART acts as dynamic time point that is used to separate the application of the DC correction from the application of the baseline correction, and the ART provides a balance between a short artifact period, from the fiducial time point, i.e., the pacing indication, to the ART, and a period following the ART that has a smaller residual voltage artifact.

In an initial step 164, the processor delineates preset boundaries of a time window within which the ART is to be computed. In an example a first boundary is set at 10 ms after the pacing indication, and a last boundary is set at 20 ms after the pacing indication, but the preset boundaries may be different in other examples.

In a first calculation step 168 the processor calculates an absolute value of a running average of means of the differences of subsets of samples up to the last window boundary. In an example the processor uses subsets of three consecutive samples, and calculates the mean of each of the differences of each of the subsets, according the following equation:

$$M(i)_{i=P}^{P+20} = \frac{1}{3} \left\| \sum_{j=1}^{3} (sig(i+j) - sig(i+j-1)) \right\| \qquad (1)$$

where P is the fiducial time point,

sig(n) is the signal value of the $n^{th}$ sample, and
M(n) is the absolute value of the mean assigned to the $n^{th}$ sample.

It will be understood that equation (1) is one method for calculating the mean, and those having ordinary skill in the art will be aware of other methods. All such methods are assumed to be included within the scope of the present disclosure.

In a second calculation step 172 the processor finds the differences between the means calculated in step 168 and the mean of the last window boundary, according to equation (2):

$$D(i)_{i=P}^{P+20} = M(i) - M(P + 20)$$

$$(2)$$

In a final step 176, the processor searches backward, from the last boundary difference for the first sample that crosses a preset threshold and that occurs after the first window boundary. In an example of the disclosure, the preset threshold is 0.5 mV/ms.

The processor uses the time of the sample found in this step as the ART.

**Baseline Correction**

In addition to the DC change described above, the pacing artifact comprises a change in the slope of the EP baseline. To compensate for this, examples of the present disclosure analyze the baseline, and in response to analysis generate a baseline fit function and a decay function. The fit function is designed to eliminate the baseline slope artifact while the decay function is designed to ensure that the fit function decreases, and eventually eliminates, the effect of the fit function with time.

Examples of the present disclosure use a fit function of the form given by equation (3), below. Equation (3) is an exponent function that decays over time, corresponding to the charge/discharge characteristic of a fixed impedance RC circuit.

$$\begin{matrix} F(n) \\ 0 < \alpha < 1 \end{matrix} = \gamma(1 - \alpha^n) \Rightarrow \begin{cases} F(0) = 0 \\ and \\ \lim_{n \to \infty} F(n) = \gamma \end{cases}$$

$$(3)$$

Where n is the sample number of the signal,

$\alpha$ is a decay rate, and
$\gamma$ is a gain.

$\alpha$ and $\gamma$ are real numbers and are selected so that F(n) is monotonic.

Examples of the present disclosure use a high-pass infinite impulse response (IIR) filter on acquired signals, and the filter, for low frequencies, effectively generates derivatives of the signal being filtered. To accommodate this, derivatives of the fit function are matched to measured derivatives of the EP signal. By performing this matching, the IIR filter does not introduce any further artifact into the signal.

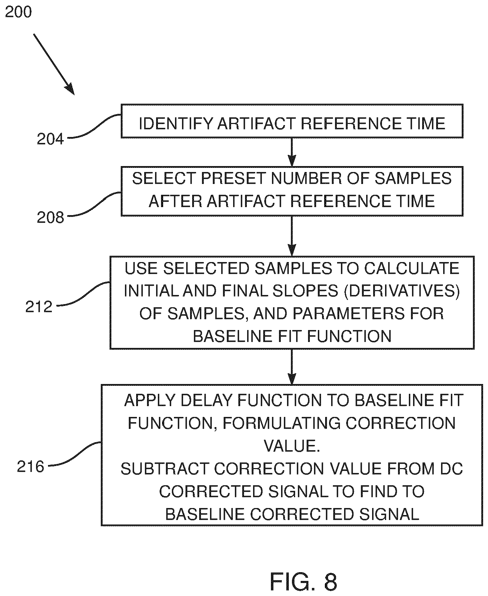

Fig. 8 is a flowchart 200 showing steps performed by processor 22 to compute the fit function, and to apply the decay function, for the baseline correction of block 104 (Fig. 4), according to an example of the present disclosure.

In an initial pacing registration step 204 the processor identifies the artifact reference time (ART) of the EP signal, as described above with reference to flowchart 160 (Fig. 7).

In a sample selection step 208, the processor selects, from the DC corrected signals received from block 100, a preset number of sample EP signals that follow the ART. In a disclosed example described herein, ten samples, s[1], s[2], ... s[10], are selected, but other examples may have more or fewer than ten samples.

In a calculation step 212, from the selected samples, the processor calculates a mean of the derivative of the first samples of the selected set, and a mean of the derivative of the last samples of the selected set. In an example there are assumed to be three first samples, having values s[1], s[2], s[3], and three last samples, having values s[8], s[9], s[10].

The first three samples have a mean derivative, herein termed K1, given by equation (4):

$$K1 \triangleq \frac{(s[3]-s[2])+(s[2]-s[1])}{2} = \frac{(s[3]-s[1])}{2}$$

$$(4)$$

The last three samples have a mean derivative, herein termed K9, given by equation (5):

$$K9 \triangleq \frac{(s[10]-s[9])+(s[9]-s[8])}{2} = \frac{(s[10]-s[8])}{2} \qquad (5)$$

Processor 22 uses the calculated values of K1, K9, to find the values of $\alpha$ and $\gamma$ in equation (3), as explained below.

From equation (3), the partial derivative of F(n) with respect to n is given by:

$$\frac{\partial F(n)}{\partial n} = \frac{\partial[\gamma(1-\alpha^n)]}{\partial n} = -\gamma\alpha^n * \text{Log}[\alpha] \qquad (6)$$

For n = 0, $\quad \dfrac{\partial F(n)}{\partial n} = -\gamma * \text{Log}[\alpha] \quad$, and this corresponds to K1, i.e.,

$$-\gamma * \text{Log}[\alpha] \triangleq K1 \qquad (7)$$

For n = 9, $\quad \dfrac{\partial F(n)}{\partial n} = -\gamma\alpha^9 * \text{Log}[\alpha] \quad$, and this corresponds to K9, i.e.,

$$-\gamma\alpha^9 * \text{Log}[\alpha] \triangleq K9 \qquad (8)$$

Equations (7) and (8) may be used to solve for $\alpha$ and $\gamma$, giving:

$$\alpha = \left(\frac{K9}{K1}\right)^{\frac{1}{9}} \qquad (9)$$

and

$$\gamma = \frac{-K1}{\text{Log}[(\frac{K9}{K1})^{1/9}]} \qquad (10)$$

In an application step 216, the processor substitutes the values of $\alpha$ and $\gamma$ calculated in equations (9) and (10), into equation (3), giving the following expression for the baseline fit function:

$$F(n) = \frac{-K1}{\text{Log}[(\frac{K9}{K1})^{1/9}]}\left(1 - \left(\frac{K9}{K1}\right)^{\frac{n}{9}}\right) \qquad (11)$$

To ensure that the baseline fit function decreases, eventually to zero for large values of n, the processor applies a decay function, D, to the fit function. The decay function prevents the EP signal from exceeding an allowable dynamic range and also prevents the signal from having baseline wander in IIR filters of the system.

In an example, the decay function D is defined according to the following equations:

$$D \triangleq a^\tau \qquad (12)$$

where a is a preset parameter, herein assumed to be 0.5, and $\tau$ is a variable time constant that depends on a value of a baseline signal $B_p$, i.e., the baseline signal for a pacing event p. $B_p$ is defined by equation (13):

$$B_p = \text{signal}_p(\text{prior}) - \text{signal}_p(\text{post}) + \text{Res}_{p-1} \qquad (13)$$

where signal$_p$(prior) is the baseline signal at a time just before the preset prior time window defined in step 128 of flowchart 120,

signal$_p$ (post) is the baseline signal at a time just after the dynamic time point, ART, found in flowchart 160, and

Res$_{p-1}$ is the residual baseline signal remaining after the (p-1) pacing estimation.

Time constant $\tau$ is set to be proportional to B$_p$, according to equation (14):

$$\tau = \frac{B_p}{K} \hspace{4cm} (14)$$

where K is a constant.

In an example, K is equal to the product of a time interval corresponding to a maximum possible rate of pacing in a clinical setup and a maximum saturation value of the ECG signal. In a disclosed example, the time interval is approximately 300 ms, and a maximum saturation value is approximately 50 mV.

The processor computes a correction value C(n), according to the following equation:

$$C(n) = (B_p - F(n)) \cdot D \hspace{4cm} (15)$$

where B$_p$ is as defined by equation (13),

F(n) is as defined by equation (11),
D is as defined by equation (12), and
n is the sample number, relative to the ART (flowchart 160) .

In step 216, to arrive at the baseline corrected signal, the processor subtracts the correction value C(n) from each sample n of the DC corrected signal.

Returning to Fig. 4, as shown in the figure, the result from baseline correction block 104, i.e., the result generated by step 216 of flowchart 200, is transferred to filtration block 108.

In block 108 processor 22 applies a filter to the signal received from baseline correction block 104. However, to avoid introducing anomalies into the signal by using the filter, the received signal is first "chopped" to detach the pacing signal, and the remaining signal is filtered, as is explained below.

In an example of the disclosure, the processor performs the chopping as follows:

The processor identifies and cuts a pacing peak from the signal received from baseline correction block 104, and stores the cut pacing peak in the memory of workstation 55.

The cut peak leaves a "hole" in the signal. The processor interpolates the region of the hole.

The processor then filters, for example using a high pass IIR filter, the signal, including the interpolated section.

After filtration, processor 22 accesses the memory to restore the cut pacing signal into the filtered signal, to produce a pacing signal with no significant artifact.

**Examples**

Example 1. A method for removal of a pacing artifact, comprising:

registering that a pacing signal has been applied to a pacing location in a heart of a patient;
positioning a first electrode in a first location in proximity to the pacing location, and coupling a first circuitry, having a first transfer function, to receive a first electrocardiogram (ECG) signal, generated in response to the pacing signal, from the first electrode;
recording a first derived ECG signal from the first circuitry in response to the first ECG signal;
positioning a second electrode in a second location in proximity to the pacing location, and coupling a second circuitry, having a second transfer function different from the first transfer function, to receive a second ECG signal, generated in response to the pacing signal, from the second electrode;
recording a second derived ECG signal from the second circuitry in response to the second ECG signal;
in response to registering the pacing signal analyzing at least one of the first and the second derived ECG signals; and
in response to the analyzing, removing the pacing artifact from the least one of the first and the second derived ECG signals.

Example 2. The method according to example 1, wherein the first circuitry and the second circuitry comprise common circuit components arranged in a common configuration.

Example 3. The method according to example 2, wherein one of the common circuit components of the first circuitry has a different electrical parameter value from the electrical parameter value of a respective component of the second circuitry.

Example 4. The method according to example 1, wherein the first circuitry and the second circuitry comprise different circuit components.

Example 5. The method according to example 1, wherein the first circuitry and the second circuitry comprise common circuit components arranged in a different configuration.

Example 6. The method according to example 1, wherein analyzing the at least one of the first and the second derived ECG signals comprises computing a direct current (DC) voltage level thereof, and wherein removing the pacing artifact comprises applying the DC voltage level as a DC correction for the at least one of the first and the second derived ECG signal.

Example 7. The method according to example 1, wherein analyzing the at least one of the first and the second derived ECG signals comprises computing a baseline fit function to compensate for a change in baseline slope of the at least one of the first and the second derived ECG signals.

Example 8. The method according to example 7, and comprising applying a decay function to the baseline fit function.

Example 9. The method according to example 1, wherein analyzing the at least one of the first and the second derived ECG signals comprises computing a bipolar ECG signal from the first and the second derived ECG signals, and wherein removing the artifact comprises removing the artifact from the bipolar ECG signal.

Example 10. The method according to example 1, wherein analyzing the at least one of the first and the second derived ECG signals comprises cutting a pacing peak therefrom so as to produce a chopped signal, interpolating and filtering the chopped signal so as to produce a filtered signal, and restoring the pacing peak to the filtered signal.

Example 11. Apparatus for removal of a pacing artifact, comprising:

a first electrode, positioned in a first location in proximity to a pacing location, in a heart of a patient, wherein a pacing signal has been applied;
a first circuitry, having a first transfer function, coupled to receive a first electrocardiogram (ECG) signal, generated in response to the pacing signal, from the first electrode;
a second electrode positioned in a second location in proximity to the pacing location;
a second circuitry, having a second transfer function different from the first transfer function, coupled to receive a second ECG signal, generated in response to the pacing signal, from the second electrode; and
a processor, configured to:

record a first derived ECG signal from the first circuitry in response to the first ECG signal,
record a second derived ECG signal from the second circuitry in response to the second ECG signal,
register that the pacing signal has been applied,
in response to registering the pacing signal, analyze at least one of the first and the second derived ECG signals, and
in response to the analyzing, remove the pacing artifact from the least one of the first and the second derived ECG signals.

The examples described above are cited by way of example, and the present disclosure is not limited by what has been particularly shown and described hereinabove. Rather the scope of the disclosure includes both combinations and subcombinations of the various features described hereinabove, as well as variations and modifications thereof which would occur to persons skilled in the art upon reading the foregoing description and which are not disclosed in the prior art.

**Claims**

1. Apparatus for removal of a pacing artifact, comprising:

a first electrode, positioned in a first location in proximity to a pacing location, in a heart of a patient, wherein a pacing signal has been applied;
a first circuitry, having a first transfer function, coupled to receive a first electrocardiogram (ECG) signal, generated in response to the pacing signal, from the first electrode;
a second electrode positioned in a second location in proximity to the pacing location;
a second circuitry, having a second transfer function different from the first transfer function, coupled to receive a second ECG signal, generated in response to the pacing signal, from the second electrode; and
a processor, configured to:

record a first derived ECG signal from the first circuitry in response to the first ECG signal,
record a second derived ECG signal from the second circuitry in response to the second ECG signal,
register that the pacing signal has been applied,
in response to registering the pacing signal, analyze at least one of the first and the second derived ECG signals, and
in response to the analyzing, remove the pacing artifact from the least one of the first and the second derived ECG signals.

2. The apparatus according to claim 1, wherein the first circuitry and the second circuitry comprise common circuit components arranged in a common configuration.

3. The apparatus according to claim 2, wherein one of the common circuit components of the first circuitry has a different electrical parameter value from the electrical parameter value of a respective component of the second circuitry.

4. The apparatus according to claim 1, wherein the first circuitry and the second circuitry comprise different circuit components.

5. The apparatus according to claim 1, wherein the first circuitry and the second circuitry comprise common circuit components arranged in a different configuration.

6. The apparatus according to claim 1, wherein analyzing the at least one of the first and the second derived ECG signals comprises computing a direct current (DC) voltage level thereof, and wherein removing the pacing artifact comprises applying the DC voltage level as a DC correction for the at least one of the first and the second derived ECG signal.

7. The apparatus according to claim 1, wherein analyzing the at least one of the first and the second derived ECG signals comprises computing a baseline fit function to compensate for a change in baseline slope of the at least one of the first and the second derived ECG signals.

8. The apparatus according to claim 7, and comprising applying a decay function to the baseline fit function.

9. The apparatus according to claim 1, wherein analyzing the at least one of the first and the second derived ECG signals comprises computing a bipolar ECG signal from the first and the second derived ECG signals, and wherein removing the artifact comprises removing the artifact from the bipolar ECG signal.

10. The apparatus according to claim 1, wherein analyzing the at least one of the first and the second derived ECG signals comprises cutting a pacing peak therefrom so as to produce a chopped signal, interpolating and filtering the chopped signal so as to produce a filtered signal, and restoring the pacing peak to the filtered signal.

FIG. 1

BALANCED SYSTEM

FIG. 2A

UNBALANCED SYSTEM

FIG. 2B

FIG. 3

FIG. 4

FIG. 5

VOLTAGE (mV)

148

152

TIME (ms)

## FIG 6

160

| DELINEATE FIRST AND LAST TIMES OF WINDOW RELATIVE TO PACING INDICATION |
164

| CALCULATE ABSOLUTE VALUE OF MEANS OF SUBSETS OF SAMPLES |
168

| FIND DIFFERENCE OF EACH SUBSET FROM LAST WINDOW SAMPLE |
172

| WORKING BACKWARDS FROM LAST WINDOW TIME, FIND FIRST TIME WHERE DIFFERENCE EXCEEDS PRESET THRESHOLD. USE THIS TIME AS ARTIFACT REFERENCE TIME |
176

## FIG. 7

200

204 — IDENTIFY ARTIFACT REFERENCE TIME

208 — SELECT PRESET NUMBER OF SAMPLES AFTER ARTIFACT REFERENCE TIME

212 — USE SELECTED SAMPLES TO CALCULATE INITIAL AND FINAL SLOPES (DERIVATIVES) OF SAMPLES, AND PARAMETERS FOR BASELINE FIT FUNCTION

216 — APPLY DELAY FUNCTION TO BASELINE FIT FUNCTION, FORMULATING CORRECTION VALUE. SUBTRACT CORRECTION VALUE FROM DC CORRECTED SIGNAL TO FIND TO BASELINE CORRECTED SIGNAL

FIG. 8

Europäisches
Patentamt

European
Patent Office

Office européen
des brevets

# EUROPEAN SEARCH REPORT

Application Number

EP 24 19 0504

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/296148 A1 (KANAGARATNAM PRAPA [GB] ET AL) 22 September 2022 (2022-09-22) * paragraphs [0053] - [0068], [0086] - [0106] * | 1-10 | INV. A61B5/346 |
| X | US 5 595 183 A (SWANSON DAVID K [US] ET AL) 21 January 1997 (1997-01-21) * column 7, line 40 - column 8, line 7 * * column 8, lines 33-40 * * column 20, line 17 - column 25, line 33 * | 1-10 | |
| X | US 2015/305640 A1 (REINKE JAMES D [US] ET AL) 29 October 2015 (2015-10-29) * paragraphs [0041] - [0043], [0047], [0048], [0056], [0063] * | 1-10 | |
| A | US 2015/148696 A1 (LALL CAROLYN ANJALI [US] ET AL) 28 May 2015 (2015-05-28) * paragraphs [0031] - [0034] * | 6-8 | |
| A | EP 0 424 607 A2 (HEWLETT PACKARD CO [US]) 2 May 1991 (1991-05-02) * column 2, line 33 - column 3, line 38 * | 6-8 | TECHNICAL FIELDS SEARCHED (IPC) A61B A61N |

The present search report has been drawn up for all claims

| Place of search | Date of completion of the search | Examiner |
|---|---|---|
| Berlin | 20 November 2024 | Trachterna, Morten |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

...........................................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 19 0504

This annex lists the patent family members relating to the patent documents cited in the above-mentioned European search report.
The members are as contained in the European Patent Office EDP file on
The European Patent Office is in no way liable for these particulars which are merely given for the purpose of information.

20-11-2024

| Patent document cited in search report | | | Publication date | Patent family member(s) | | Publication date |
|---|---|---|---|---|---|---|
| US 2022296148 | A1 | | 22-09-2022 | EP | 4013293 A1 | 22-06-2022 |
| | | | | US | 2022296148 A1 | 22-09-2022 |
| | | | | WO | 2021028699 A1 | 18-02-2021 |
| US 5595183 | A | | 21-01-1997 | AT | E201974 T1 | 15-06-2001 |
| | | | | CA | 2213216 A1 | 22-08-1996 |
| | | | | DE | 69613350 T2 | 25-04-2002 |
| | | | | EP | 0809464 A1 | 03-12-1997 |
| | | | | ES | 2159725 T3 | 16-10-2001 |
| | | | | JP | 3677780 B2 | 03-08-2005 |
| | | | | JP | H11500037 A | 06-01-1999 |
| | | | | US | 5595183 A | 21-01-1997 |
| | | | | WO | 9625097 A1 | 22-08-1996 |
| US 2015305640 | A1 | | 29-10-2015 | CN | 106231994 A | 14-12-2016 |
| | | | | EP | 3134175 A1 | 01-03-2017 |
| | | | | JP | 6533569 B2 | 19-06-2019 |
| | | | | JP | 2017518846 A | 13-07-2017 |
| | | | | US | 2015305640 A1 | 29-10-2015 |
| | | | | WO | 2015164167 A1 | 29-10-2015 |
| US 2015148696 | A1 | | 28-05-2015 | US | 2015148696 A1 | 28-05-2015 |
| | | | | WO | 2013162613 A1 | 31-10-2013 |
| EP 0424607 | A2 | | 02-05-1991 | DE | 69018924 T2 | 21-12-1995 |
| | | | | EP | 0424607 A2 | 02-05-1991 |
| | | | | JP | 2995080 B2 | 27-12-1999 |
| | | | | JP | H03151934 A | 28-06-1991 |
| | | | | US | 5033473 A | 23-07-1991 |

EPO FORM P0459

For more details about this annex : see Official Journal of the European Patent Office, No. 12/82

**EP 4 497 387 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63528709
- US 5539199 A
- US 5443489 A
- US 5558091 A
- US 6172499 B
- US 6239724 B
- US 6332089 B
- US 6484118 B
- US 6618612 B
- US 6690963 B
- US 6788967 B
- US 6892091 B
- US 7536218 B
- US 7756576 B
- US 7848787 B
- US 7869865 B
- US 8456182 B